# EUROPEAN PATENT SPECIFICATION

(11) **EP 2 401 958 B1**
(45) Date of publication and mention of the grant of the patent: **06.03.2013**
(21) Application number: 11171262.6
(22) Date of filing: 24.06.2011
(51) Int. Cl.: A61B 5/00, A61B 1/24

(54) **Intraoral observation equipment**
Intraorales Betrachtungsgerät
Équipement d'observation intra-orale

(30) Priority: 02.07.2010 JP 2010152418
(43) Date of publication of application: 04.01.2012
(73) Proprietor: Inspektor Research Systems B.V., NL-1072 XX Amsterdam (NL); GC Corporation, Bunkyo-ku Tokyo 113-0033 (JP)
(72) Inventor: de Jong, Elbert de Josselin, 1406 LL Bussum (NL); Inaba, Daisuke, Iwate 020-8505 (JP); Takada, Mitsuaki, Tokyo 174-8585 (JP); Shinjo, Takao, Tokyo 174-8585 (JP)
(74) Representative: Smart, Peter John

(56) References cited:
- US-A1- 2006 020 169
- US-A1- 2008 118 886
- US-A1- 2009 181 339
- WALSH, LAURENCE J.; SHAKIBAIE, FARDAD: "Ultraviolet-induced fluorescence: shedding new light on dental biofilms and dental caries", AUSTRALASIAN DENTAL PRACTICE, vol. 18, no. 6, 2007, pages 56-60, XP002660218, Cammeray, NSW ISSN: 1445-5269

## Description

### BACKGROUND OF THE INVENTION

### Field of the Invention

The present invention relates to an intraoral observation equipment used for a dental treatment and more particularly to an intraoral observation equipment capable of discriminating an abnormal portion, in which a gingivitis and a curing resin exist, from a normal portion.

### Description of the Related Art

A special knowledge and experience are necessary to discriminate a portion, in which a so-called periodontal disease such as a gingivitis and the like exists, and a portion of a tooth, in which a resin is buried with treatment, by naked eyes. Accordingly, even when an image, which is ordinarily picked up from inside of the oral cavity of a patient, is shown to the patient, the patient may not have an actual feeling of the image, and it may be difficult for a dentist to definitely explain the image.

It is known that the portion of a periodontal disease caused in an oral cavity and the portion of a resin buried in a tooth (which may be described as "diseased portion and the like" hereinafter) have such a property that when a light having a peak value in a wavelength region of 360 nm to 420 nm is radiated to the oral cavity, the portions emit a fluorescent light having a wavelength of 450 nm or more different from the radiated light. A QLF method (Quantitative Light-Induced Fluorescence) for quantitatively finding a diseased portion and the like making use of the property is known.

For example, Japanese Patent Application Laid-Open (JP-A) No. 2006-174977 also discloses a dental inspection equipment for quantitatively evaluating a diseased portion and the like using the QLF method. Further, JP-A No. 2004-65994 also discloses a dental device to which the QLF method is applied to inspect a tooth surface for the purpose of discriminating a diseased portion.

Document US 2009/0181339 A1 discloses an indexoral camera in accordance with the preamble of claim 1.

### Citation List

### Patent Literature

Patent Document 1: Japanese Patent Application Laid-Open No. 2006-174977
Patent Document 2: Japanese Patent Application Laid-Open No. 2004-065994

However, since an image in an oral cavity, which is obtained by the QLF method including the conventional technologies, is very dark and has a color greatly different from an actual color in the oral cavity, a patient to whom the image is explained considerably feels strange as to the image.

In view of the problems, an object of the invention is to provide a dental intraoral observation equipment capable of obtaining a sharp and easily observable image even in the QLF method.

### SUMMARY OF THE INVENTION

The invention will be explained below. Although reference symbols in accompanying drawings are shown in parentheses for the purpose of easy understanding, the invention is not limited to an embodiment shown in the drawings.

The invention according to a first aspect is an intraoral observation equipment (10) which includes a light source unit (11) including a white light-emitting diode light source (13) and a light-emitting diode light source (14) for producing fluorescence having a peak within a wavelength range of 360 nm to 420 nm, an image taking device (17) which receives a reflected light and a fluorescent light of the lights emitted from the light source unit and which converts those lights into electrical signals, a tuning means (15, 16) which is arranged between the light source unit and the image taking device and which shields against or damps a light having a wavelength component of 420 nm or less and which damps the amount of light having a wavelength component of 450-600 nm; and a signal treating means (18) configured to treat the electrical signals converted by the image taking device, wherein, in a normal observation mode where only the white light-emitting diode light source is turned on, the signal treating means modifies the received light signals in accordance with predetermined relations, and in a QLF mode where only the light-emitting diode light source for producing fluorescence is turned on, the signal treating means modifies the received light signals in accordance with other predetermined relations.

Here, the phrase "a tuning means is arranged between the light source unit and the image taking device" means that the position of the turning means is between the light source unit and the image taking device. It also means that although the position of the turning means is not between the light source unit and the image taking device, but the position is optically between the light source unit and the image taking device by the light-path control using reflector, prism, and so on. Hereinafter, the phrase means the same.

The invention according to a second aspect is the intraoral observation equipment (10) according to claim 1, wherein the tuning means has a first tuning means (15) which is arranged between the light source unit (11) and the image taking device (17) and which shields against or damps a light having a wavelength component of 420 nm or less, and wherein the tuning means has a second tuning means (16) which is arranged between the light source unit and the image taking device and which damps the amount of light having a wavelength component of 450-600 nm.

The invention according to a third aspect is the intraoral observation equipment according to the first aspect or the second aspect, wherein the first tuning means 15 and the second tuning means 16 are optical filters.

The invention according to a fourth aspect is an intraoral observation equipment which includes a light source unit including a white light-emitting diode light source and a light-emitting diode light source for producing fluorescence having a peak within a wavelength range of 360 to 420 nm; an image taking device which receives a reflected light and a fluorescent light of the lights emitted from the light source unit and which converts those lights into electrical signals; a first tuning means which is arranged between the light source unit and the image taking device and which shields against or damps a light having a wavelength component of 420 nm or less; a second tuning means which damps and memorizes the electrical signals of light having a wavelength component of 450 to 600 nm which has been received and converted into electrical signals by the image taking device; and a signal treating means configured to treat the electrical signals converted by the image taking device and the electrical signals memorized by the second tuning means, wherein, in a normal observation mode where only the white light-emitting diode light source is turned on, the signal treating means modifies the electrical signals in accordance with predetermined relations, and in a QLF mode where only the light-emitting diode light source for producing fluorescence is turned on, the signal treating means modifies the electrical signals in accordance with other predetermined relations.

According to the invention, in a QLF observation, the amount of light of an entire wavelength region (RGB) can get an approximately uniform white balance by shielding against a light generated from the light source for producing fluorescence, greatly damping a fluorescent light produced in a green region, and transmitting a light in a red wavelength region in a large amount by the wavelength tuning means. A sharp and easily observable image in which a recognition property is greatly improved can be obtained by setting the white balance to an appropriate value.

### BRIEF DESCRIPTION OF THE DRAWINGS

FIG. 1 is a conceptual view of an intraoral observation equipment according to an embodiment;
FIG. 2 is a view of the intraoral observation equipment of FIG. 1 when viewed from a right side of the sheet; and
FIGS. 3A and 3B show treated images for explaining results of an example and a comparative example.

### DESCRIPTION OF THE PREFERRED EMBODIMENTS

An operation and a merit of the invention will be apparent from an embodiment described below. The invention will be explained below based on the embodiment shown in the drawings. However, the invention is not limited to the embodiment.

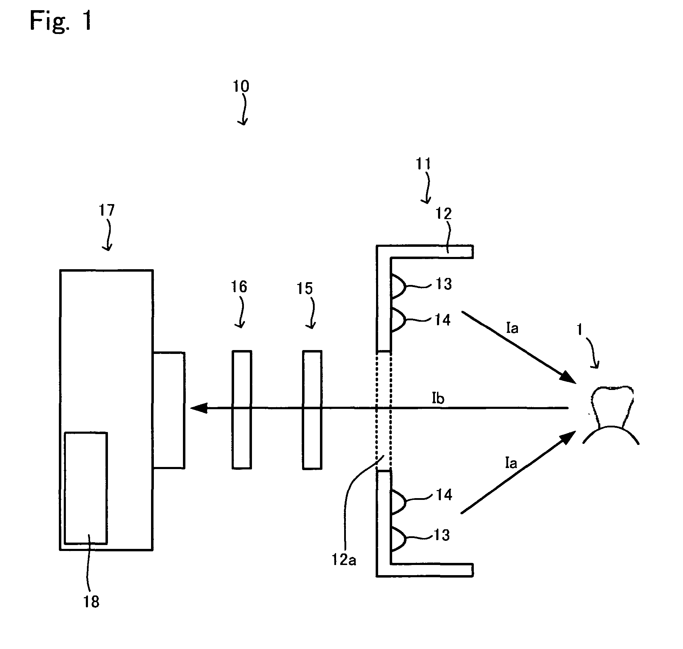

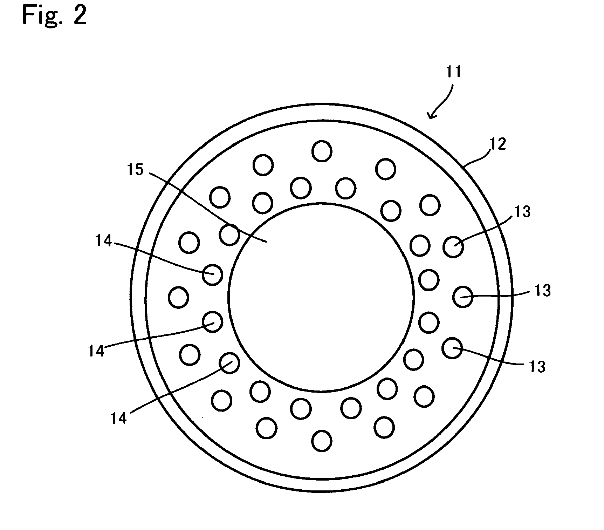

FIG. 1 is a view conceptually showing an intraoral observation equipment 10 according to the embodiment. FIG. 2 is a view of the intraoral observation equipment 10 of FIG. 1 when viewed from a right side of the sheet.
The intraoral observation equipment 10 includes a light source unit 11, wavelength tuning means 15, 16, an image taking means 17, and a signal treating means 18.

As apparent from FIGS. 1 and 2, the light source unit 11 includes a hood 12, a white light source 13, and a light source 14 for producing fluorescence.
The hood 12 is a bottomed cylindrical member, and a circular hole 12a is formed to a bottom plate about a cylindrical axis center. A light reaches the wavelength tuning means 15, 16 and the image taking means 17 which are described later via the hole 12a. The hood 12 functions as a so-called light guide means for efficiently guiding lights emitted from the white light source 13 and the light source 14 for producing fluorescence into an oral cavity. In addition to the above-mentioned function, the hood 12 can greatly reduce the amount of lights which directly enter the eyes of a patient and a practitioner from the white light source 13 and the light source 14 for producing fluorescence.

The white light source 13 is a light source which emits a light having a wavelength that covers the entire region of at least a visible light. The white light source 13 is an illumination light source used to observe an ordinary oral cavity. Accordingly, although the type of the white light source 13 is not particularly limited, the white light source 13 is preferably a white light emitting diode (white LED) from a view point of a life, a brightness, and a power consumption of the light source.

The light source 14 for producing fluorescence is a light source for radiating a light having a specific wavelength into an oral cavity. The specific wavelength means a wavelength that allows a diseased portion and the like to emit a fluorescent light when a light having the wavelength is radiated to the portion and the like. For example, a light, which has a peak intensity for radiating a light having a peak intensity within a wavelength region of, for example, 360 nm to 420 nm, can be exemplified. Although the type of the light source is not particularly limited, the light source is preferably a light emitting diode (LED) having a peak intensity within the wavelength region from a view point of a life, a brightness, and a power consumption of the light source. That is, the light source is an LED that emits a violet light visually.

As apparent from FIGS. 1 and 2, the white light sources 13 and the light sources 14 for producing fluorescence are disposed on the bottom portion of the hood 12 around a circumference about the cylindrical axis center of the hood 12 at predetermined intervals. With this arrangement, it is possible to uniformly illuminate inside of the oral cavity. In addition to the above mentioned, it is preferable that the white light sources 13 and the light sources 14 for producing fluorescence be disposed at the positions where a non-shade effect can be obtained so that a picked-up image is not shaded by the shades resulting from the positions of the light sources.

The light source unit 11 can select and switch a normal observation mode in which the white light sources 13 are turn on and the light sources 14 for producing fluorescence are turned off and a QLF mode in which the white light sources 13 are turned off and the light sources 14 for producing fluorescence are turned on inversely. The light sources may be switched manually as described later or may be automatically switched depending on the number of times at which a shutter is operated. Further, the light sources may be switched optionally by a setting of the observation equipment.

Next, the wavelength tuning means 15, 16 will be explained. The wavelength tuning means 15, 16 are means for shielding against or damping the amount of light within a predetermined wavelength range. The embodiment includes the first tuning means 15 and the second tuning means 16.

The first tuning means 15 is a means for shielding against or damping a light to be transmitted therethrough when the light has a wavelength component of 420 nm or less and transmitting the light when the light has a wavelength component other than the above wavelength component without damping the light. The first tuning means 15 is not particularly limited as long as it can tune the wavelength as described above. The embodiment exemplifies an optical filter as a preferable mode from a view point of versatility and availability. Further, a surface treatment is preferably performed on the hood 12 side of the first tuning means 15 for antifogging. This is because the surface is liable to be fogged by a breath directly blown onto the surface.

The first tuning means 15 is disposed to close the hole 12a of the hood 12. More specifically, the first tuning means 15 can tune the wavelength of a light which is captured to the image taking device 17 from inside of the oral cavity. A specific operation of the first tuning means 15 will be explained later in detail.

The second tuning means 16 is a means for greatly restricting transmission of a light to be transmitted therethrough, in particular transmission of a light having a green wavelength region which is a fluorescence producing component, specifically transmission of a light having a wavelength component of 450 nm to 600 nm and for damping the wavelength component. The damping causes the damped wavelength component (green), the wavelength component (blue) weakened by the first tuning means 15, and the wavelength component (red) whose amount is small in the light emitting components to approximate the wavelength distribution of the respective wavelength components (red, blue, green) of the light received by the image taking device 17 under the normal light source. With the operation, the amount of light of an entire wavelength region (RGB) can be provided with an approximately uniform white balance.
Accordingly, the second tuning means 16 transmits the wavelength components other than the above wavelength components therethrough without damping them. The second tuning means 16 is not particularly limited as long as it can tune the wavelengths as described above. In the embodiment, an optical filter is preferably used from a view point of versatility and availability.

Further, the second tuningmeans maybe composed of a signal treating means for recording the light signals, which are received by the image taking device 17 and converted to electrical signals after the signals corresponding to the wavelength component are electrically damped in addition to the second tuning means composed of the optical filter.

The second tuning means 16 is disposed on the image taking device 17 side of the first tuningmeans 15 so as to be superimposed to the first tuning means 15. More specifically, the second tuning means 16 can tune the wavelength of a light, which is captured to the image taking device 17 from inside of the oral cavity. To minimize an optical color difference, the first tuning means 15 is preferably disposed in intimate contact with the second tuning means 16. However, the first tuning means 15 and the second tuning means 16 are not limited to the above disposition. Specific operations of the first tuning means 15 and the second tuning means 16 will be explained later in detail.

In the above embodiment of the invention, an example having two tuning means (i.e. the first tuning means and the second tuning means) has been described. However, the invention is not limited to the embodiment as long as the invention is a mode which is provided with tuning means so that the tuning means can attenuate or interrupt the above wavelength range. In other words, the mode may comprise more than two tuning means.

Next, the image taking device 17 will be explained. The image taking device 17 is a device for receiving the light from the oral cavity which is transmitted through the wavelength tuning means 15, 16 and converting the light to the electrical signals of respective pixels. More specifically, it is sufficient that the image taking device 17 includes a light receiving element such as a CCD, and the image taking device 17 is not particularly limited as long as it has the above arrangement. A digital still camera, for example, can be used as the image taking device 17.

The signal treating means 18 is a means which subjects the electrical signals of images obtained by the image taking device 17 to a predetermined treatment and outputs the electrical signals as the images. Accordingly, the signal treating means 18 includes, for example, an input port to which the signals from the image taking device 17 are input, a memory device (ROM) to which necessary information of a predetermined arithmetic expression is previously recorded, a central operation unit (CPU) for executing an arithmetic operation, a RAM functioning as a work area and a temporary information memorizing region, and an output port to which a result of arithmetic operation is output.

The signal treating means 18 of the embodiment is configured to execute a treatment to the normal observation mode, in which the white light sources 13 are turned on and the light sources 14 for producing fluorescence are turned off, and a treatment to the QLF mode, in which the white light sources 13 are turned off and the light sources 14 for producing fluorescence are turned on, respectively. The treatments executed by the signal treating means 18 will be explained later in detail together with an explanation of the intraoral observation equipment 10.

In the embodiment, the signal treating means 18 is contained in the image taking device 17. With the configuration, the intraoral observation equipment 10 can be made compact and can be used easily. However, the signal treating means 18 is not prevented from being disposed separately from the image taking device 17 and further may be connected to the image taking device 17 from the outside.

Further, the image taking device 17 may include a power supply for operating the light source unit 11, the image taking device 17, and the signal treating means 18. With the configuration, the equipment has improved portability and can be easily operated.

Next, the operation of the intraoral observation equipment 10 will be explained referring to FIG. 1.

When the normal observation of inside of the oral cavity is executed by the intraoral observation equipment 10, the inside of the oral cavity is observed in the normal observation mode. At the time, the white light sources 13 are turned on and the light sources 14 for producing fluorescence are turned off.

The lights from the white light sources 13 are radiated to teeth and gums as shown by Ia, Ia of FIG. 1. A light reflected from the teeth and gums reaches the image taking device 17 as shown by Ib in FIG. 1. That is, the reflected light passes through the hole 12a of the hood 12 and is transmitted through the first tuning means 15. At the time, a reflected light having a wavelength component of 420 nm or less is shielded against or damped and a reflected light having a wavelength component other than the above wavelength component is transmitted without being damped. Further, the reflected light is transmitted through the second tuning means 16. At the time, a reflected light having a wavelength component within a range of 450 nm to 600 nm is transmitted after it is damped, and a reflected light having a wavelength component other than the above wavelength component is transmitted without being damped. The image taking device 17 receives the transmitted light, and the transmitted light is converted to the electrical signals. Accordingly, at the time, the image taking device 17 receives the reflected light having strong red (R) color.

The signal treating means 18 selects the treatment of the normal observation mode corresponding to the normal observation mode and executes the treatment to the received light signals. Specifically, it is sufficient for the signal treating means 18 to execute a correction for modifying the wavelength components which are shielded against and damped based on the received light signals. That is, relations are previously memorized in the signal treating means 18 so as to correspond to the above characteristics of the first tuning means 15 and the second tuning means 16 in use, and the received light signals are modified based on the relations. With the operation, the inside of the oral cavity can be observed in a color tone which is the same as that when the inside of the oral cavity is observed under an ordinary white light.

In contrast, when a QLF observation is executed in the oral cavity by the intraoral observation equipment 10, the inside of the oral cavity is observed in the QLF mode. At the time, the light sources 14 for producing fluorescence are turned on, and the white light sources 13 are turned off.

The lights from the light sources 14 for producing fluorescence are radiated to the teeth and gums as shown in Ia, Ia of FIG. 1 likewise the case of the white light sources 13. In the QLF mode, fluorescent lights, which have a wavelength different from that of a light radiated from the diseased portion and the like of the teeth and gums, are produced according to the property of the light sources. The fluorescent lights have a wavelength of a green component and specifically are lights having a wavelength of 450 nm to 600 nm. Accordingly, a light reflected from the teeth and gums and a fluorescent light from the diseased portion and the like reach the image taking device 17 as shown by Ib in FIG. 1. At the time, the reflected light and the fluorescent light pass through the hole 12a of the hood 12 and are transmitted through the first tuning means 15. At the time, a reflected light and a fluorescent light having a wavelength component of 420 nm or less are shielded or damped and a reflected light and a fluorescent light having a wavelength component other than the above wavelength component are transmitted without being damped. Since the lights from the light sources 14 for producing fluorescence originally have a peak intensity within a wavelength region of 360 nm to 420 nm, almost all the reflected light is shielded against or damped by the first tuning means 15.

Further, the reflected light and the fluorescent light are transmitted through the second tuning means 16. At the time, a reflected light and a fluorescent light having a wavelength component within the range of 450 nm to 600 nm are transmitted after they are damped, and a reflected light and a fluorescent light having a wavelength component other than the above wavelength component are transmitted without being damped. More specifically, the second tuning means 16 greatly damps the fluorescent light.

Accordingly, the ratio of a light component in a visible light region can be made approximately the same as the ratio of a light component obtained from a normal light source by the shielding or the damping executed by the first tuning means 15 described above, the damping executed by the second tuning means 16, and the transmission of the light having a wavelength component larger than 600 nm.
More specifically, since a light having a wavelength of 420 nm or less is shielded against, a light in a wavelength range of 450 nm to 600 nm is damped, and a light having a wavelength range larger than 600 nm is not almost included originally, a light, which has a wavelength distribution similar to that of a light obtained from the normal light source having a low light amount level, is received by the image taking device 17 in the entire wavelength range, and the light is converted to the electrical signals.

The signal treating means 18 selects the treatment of the QLF mode corresponding to the QLF mode and executes the treatment to the received light signals. Specifically, it is sufficient for the signal treating means 18 to execute a correction for modifying the tuned wavelength components based on the received light signals. More specifically, since the light amount level of a specific wavelength region is tuned using the first tuning means 15 and the second tuning means 16 in use, an image, which has a white balance similar to that under the ordinary light source, can be obtained by fine tuning. As a result, a QLF image, in which a diseased portion and the like are shown by an apparently separated color, can be obtained regardless that the inside of the oral cavity has a color tone near to a natural color in its entirety. For the purpose, the relations among the light source of the light source 14 for producing fluorescence, the first tuning means 15, and the second tuning means 16 are previously memorized in the signal treating means 18 based on the characteristics of the light sources of the light sources 14 for producing fluorescence, the first tuning means 15, and the second tuning means 16, thereby modifying the received electrical signals.

In the observation of an oral cavity executed using a conventional QLF, since a wavelength tuning means has been not used or the oral cavity has been observed by extracting only a fluorescent light by shielding against a light having a wavelength component of 420 nm or less, an image of the oral cavity has been dark or the image has had a color tone greatly different from that of an actual oral cavity. When a dentist explains the oral cavity while showing the image to a patient, a problem has arisen in the color tone. However, the invention can solve the problems.

Further, when a conventional image is corrected to have a normal color tone by an image treatment, an expensive image treatment device has been necessary. In contrast, according to the invention, the color tone can be corrected by a relatively simple device.

The normal mode and the QLF mode may be switched by a changeover switch disposed to, for example, the image taking device or maybe automatically switched sequentially by operating a shutter for imaging.

### [Example]

In an example, the inside of an oral cavity was observed in the QLF mode of the intraoral observation equipment 10, and an image of the oral cavity was obtained. The equipment was specifically configured as described below.
- Light source for producing fluorescence: LED having peak wavelength of 400 nm;
- First tuning means: optical sharp cut filter for shielding against a light having a wavelength component of 420 nm or less;
- Second tuning means: optical filter for damping a light having a wavelength component of 450 nm to 600 nm;
- Image taking device: digital camera (digital camera GXR A12 made by Ricoh Company, Ltd, 50 mm camera unit); and
- Signal treating means: firmware in digital camera made by Ricoh Company, Ltd

In contrast, as an comparative example, imaging was executed in a QLF method using a conventional device (QLF Inspektor Pro system by Inspector Research Systems (market entry 2004)).

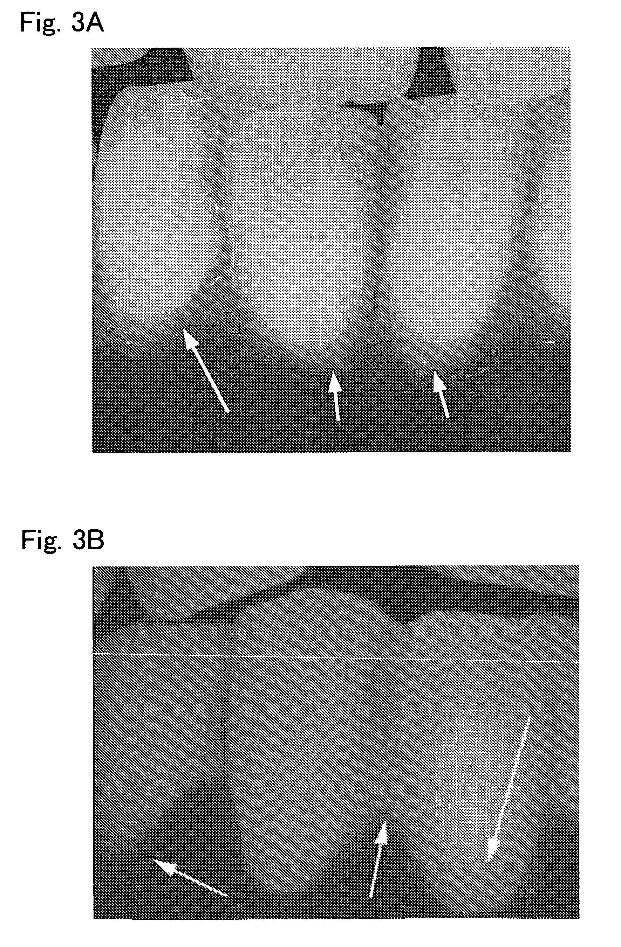

FIGS. 3A and 3B show images obtained by the example and the comparative example. FIG. 3A shows the image obtained by the example and FIG. 3B shows the image obtained by the comparative example. In each of FIGS. 3A and 3B, portions where gingivitis occurs are shown by arrows. As apparent from FIGS. 3A and 3B, according to the invention, a bright image near to a natural color can be obtained.

The invention has been explained above as to the embodiment which is supposed to be practically employed as well as preferable at present. However, it should be understood that the invention is not limited to the embodiment disclosed in the specification and can be appropriately modified within the range which does not depart from the invention, which can be read from the appended claims.

### Description of the Reference Numerals

- 10: intraoral observation equipment
- 11: light source unit
- 12: hood
- 13: white light source
- 14: light source for producing fluorescence
- 15: first tuning means
- 16: second tuning means
- 17: image taking means
- 18: signal treating means

## Claims

1. An intraoral observation equipment comprising:
a light source unit comprising a white light-emitting diode light source and a light-emitting diode light source for producing fluorescence having a peak within a wavelength range of 360-420 nm;
an image taking device configured to receive a reflected light and a fluorescent light of the lights emitted from the light source unit and which converts those lights into electrical signals; and **characterised by**
a tuning means which is arranged between the light source unit and the image taking device and configured to shield against or damp a light having a wavelength component of 420 nm or less and configured to damp the amount of light having a wavelength component of 450-600 nm; and
a signal treating means configured to treat the electrical signals converted by the image taking device,
wherein, in a normal observation mode where only the white light-emitting diode light source is turned on, the signal treating means is configured to modify the electrical signals in accordance with predetermined relations, and
in a QLF mode where only the light-emitting diode light source for producing fluorescence is turned on, the signal treating means is configured to modify the electrical signals in accordance with other predetermined relations.

2. The intraoral observation equipment according to claim 1, wherein the tuning means has a first tuning means which is arrangedbetween the light source unit and the image taking device and is configured to which shield against or damp a light having a wavelength component of 420 nm or less, and wherein the tuning means has a second tuning means which is arranged between the light source unit and the image taking device and which is configured to damp the amount of light having a wavelength component of 450-600 nm.

3. The intraoral observation equipment according to claim 1 or 2, wherein the first tuning means and the second tuning means are optical filters.

4. The intraoral observation equipment according to claim 1, wherein the tuning means comprises:
a first tuning means which is arranged between the light source unit and the image taking device and which is configured to shield against or damp a light having a wavelength component of 420 nm or less; and
a second tuning means which is configured to damp and memorize the electrical signals of light having a wavelength component of 450-600 nm which has been received and converted into electrical signals by the image taking device, and wherein the signal treating means is configured to treat the electrical signals converted by the image taking device and the electrical signals memorized by the second tuning means.

## Patentansprüche

1. Eine intraorale Betrachtungsvorrichtung umfassend:
eine Lichtquelleneinheit umfassend eine Weißlicht-Leuchtdiodenlichtquelle und eine Leuchtdiodenlichtquelle zum Erzeugen von Fluoreszenz mit einer Spitze innerhalb eines Wellenlängenbereichs von 360-420 nm;
eine Bildaufnahmevorrichtung ausgelegt zum Empfangen eines reflektierten Lichts und eines fluoreszierenden Lichts von den von der Lichtquelleneinheit emittierten Lichtern und welche diese Lichter in elektrische Signale umwandelt; und **gekennzeichnet, durch**
eine Einstellvorrichtung, welche zwischen der Lichtquelleneinheit und der Bildaufnahmevorrichtung angeordnet ist, und ausgelegt ist zum Abschirmen gegen oder Abschwächen von Licht mit einem Wellenlängenanteil von 420 nm oder weniger und ausgelegt ist zum Abschwächen der Menge von Licht mit einem Wellenlängenanteil von 450-600 nm; und
einer Signalverarbeitungseinrichtung ausgelegt zum Verarbeiten der durch die Bildaufnahmevorrichtung umgewandelten elektrischen Signale,
wobei in einem normalen Betrachtungsmodus, in welchem nur die Weißlicht-Leuchtdiodenlichtquelle angeschaltet ist, die Signalverarbeitungseinrichtung ausgelegt ist zum Ändern der elektrischen Signale in Übereinstimmung mit vorgegebenen Beziehungen, und
in einem QLF-Modus, in welchem nur die Leuchtdiodenlichtquelle zum Erzeugen von Fluoreszenz angeschaltet ist, die Signalverarbeitungseinrichtung ausgelegt ist zum Ändern der elektrischen Signale in Übereinstimmung mit anderen vorgegebenen Beziehungen.

2. Die intraorale Betrachtungsvorrichtung gemäß Anspruch 1, wobei die Einstellvorrichtung eine erste Einstellvorrichtung aufweist, welche zwischen der Lichtquelleneinheit und der Bildaufnahmevorrichtung angeordnet ist und welche ausgelegt ist zum Abschirmen gegen oder Abschwächen von einem Licht mit einem Wellenlängenanteil von 420 nm oder weniger, und wobei die Einstellvorrichtung eine zweite Einstellvorrichtung aufweist, welche zwischen der Lichtquelleneinheit und der Bildaufnahmevorrichtung angeordnet ist und welche ausgelegt ist zum Abschwächen der Menge des Lichts mit einem Wellenlängenanteil von 450-600 nm.

3. Die intraorale Betrachtungsvorrichtung gemäß Anspruch 1 oder 2, wobei die erste Einstellvorrichtung und die zweite Einstellvorrichtung optische Filter sind.

4. Die intraorale Betrachtungsvorrichtung gemäß Anspruch 1, wobei die Einstellvorrichtung umfasst:
eine erste Einstellvorrichtung, welche zwischen der Lichtquelleneinheit und der Bildaufnahmevorrichtung angeordnet ist und welche ausgelegt ist zum Abschirmen gegen oder Abschwächen von Licht mit einem Wellenlängenanteil von 420 nm oder weniger; und
einer zweiten Einstellvorrichtung, welche ausgelegt ist zum Abschwächen oder Speichern der elektrischen Signale des Lichts mit einem Wellenlängenanteil von 450-600 nm, die durch die Bildaufnahmevorrichtung empfangen und in elektrische Signale umgewandelt wurden, und wobei die Signalverarbeitungseinrichtung ausgelegt ist zum Verarbeiten der durch die Bildaufnahmevorrichtung umgewandelten elektrischen Signale und der durch die zweite Einstellvorrichtung gespeicherten elektrischen Signale.

## Revendications

1. Equipement d'observation intra-orale comprenant :
une unité de source de lumière comprenant une source de lumière à diode émettant une lumière blanche et une source de lumière à diode émettant de la lumière pour produire une fluorescence ayant un pic dans une plage de longueurs d'ondes de 360 à 420 nm ;
un dispositif de prise d'image configuré pour recevoir une lumière réfléchie et une lumière fluorescente des lumières émises de l'unité de source de lumière et qui convertit ces lumières en signaux électriques ; et **caractérisé par**
un moyen de réglage qui est disposé entre l'unité de source de lumière et le dispositif de prise d'image et configuré pour faire écran contre ou atténuer une lumière ayant un composant de longueur d'onde de 420 nm ou inférieur et configuré pour atténuer la quantité de lumière ayant un composant de longueur d'onde de 450 à 600 nm ; et
un moyen de traitement de signal configuré pour traiter les signaux électriques convertis par le dispositif de prise d'image,
dans lequel, dans un mode d'observation normal dans lequel seule la source de lumière à diode émettant une lumière blanche est allumée, le moyen de traitement de signal est configuré pour modifier les signaux électrique conformément aux relations prédéterminées, et
dans un mode QLF dans lequel seule la source de lumière à diode émettant de la lumière pour produire une fluorescence est allumée, le moyen de traitement de signal est configuré pour modifier les signaux électriques conformément à d'autres relations prédéterminées.

2. Equipement d'observation intra-oral selon la revendication 1, dans lequel le moyen de réglage a un premier moyen de réglage qui est disposé entre l'unité de source de lumière et le dispositif de prise d'image et qui est configuré pour faire écran contre ou atténuer une lumière ayant un composant de longueur d'onde de 420 nm ou inférieur, et dans lequel le moyen de réglage a un deuxième moyen de réglage qui est disposé entre l'unité de source de lumière et le dispositif de prise d'image et qui est configuré pour atténuer la quantité de lumière ayant un composant de longueur d'onde de 450 à 600 nm.

3. Equipement d'observation intra-oral selon la revendication 1 ou 2, dans lequel le premier moyen de réglage et le deuxième moyen de réglage sont des filtres optiques.

4. Equipement d'observation intra-oral selon la revendication 1, dans lequel le moyen de réglage comprend :
un premier moyen de réglage qui est disposé entre l'unité de source de lumière et le dispositif de prise d'image et qui est configuré pour faire écran contre ou atténuer une lumière ayant un composant de longueur d'onde de 420 nm ou inférieur ; et
un deuxième moyen de réglage qui est configuré pour atténuer et mémoriser les signaux électriques de lumière ayant un composant de longueur d'onde de 450 à 600 nm qui a été reçu et converti en signaux électriques par le dispositif de prise d'image, et dans lequel le moyen de traitement de signal est configuré pour traiter les signaux électriques convertis par le dispositif de prise d'image et les signaux électriques mémorisés par le deuxième moyen de réglage.
